# EUROPEAN PATENT APPLICATION

(11) **EP 0 854 130 A1**
(43) Date of publication of application: **22.07.1998**
(21) Application number: 98200002.8
(22) Date of filing: 02.01.1998
(51) Int. Cl.: C07C 67/03, C07C 69/76

(54) **Recovery of monomers from polyethylene naphthalate**

(30) Priority: 15.01.1997 US 784169
(71) Applicant: EASTMAN KODAK COMPANY, Rochester, New York 14650 (US)
(72) Inventor: Naujokas, Andrius, Rochester, New York 14650-2201 (US)
(74) Representative: Parent, Yves

(57) **Abstract**

There is described a process for depolymerizing polyethylene naphthalate polyester to monomer components which then can be used to make virgin polyester.

## Description

This invention relates to a process for recovering ester and glycol components from polyethylene naphthalate.

Polyethylene naphthalate, which also is called polyethylene 2,6 naphthalene dicarboxylate polyesters and is abbreviated as PEN, has outstanding physical properties for a number of uses, such as in textile, film and other applications. It has been found to be particularly useful as a film base for magnetic recording materials and the photographic industry has started to use it as a film support in new photographic products. PEN, as well as its precursor dimethyl 2,6 naphthalene dicarboxylate, abbreviated as NDC, is more costly than other polyesters, such as polyethylene terephthalate, that have been used in these products. The polymer is a linear polyester produced from ethylene glycol and dimethyl 2,6 naphthalene dicarboxylate (NDC) using conventional polymerization processes. Due to relatively high cost of the polyester and to minimize the need for disposal it is desirable to recycle and reuse waste PEN.

Two distinctly different routes have been used to recycle polyesters in general. The first route is direct recycle. This can be done when the polyester scrap is completely clean or can be cleaned by washing or other means. The clean(ed) polyester is used in place of virgin polyester at the appropriate point in the manufacturing process. Depending upon the manufacturing steps in the process, degradation of the polyester can occur during such steps as melting and extrusion due to thermal and shear effects. This results in a deterioration of the physical properties of the polyester, such as a lowering of molecular weight. Generally for high grade products, such as film base, recycle polyester is blended in limited amounts with virgin polyester. Since PEN have relatively high melting and softening temperatures significant degradation of the polyester can be expected. Furthermore, if the polyester scrap is contaminated, or is not thoroughly cleaned, additional degradation of properties can occur.

The second route is depolymerization of the polyester to component monomers, recovering the monomers and then using them to produce new polyester. This route has been used for recovery of monomers from polyethylene terephthalate polyester, abbreviated as PET. Recovery techniques are described, for example in US-A-5,414,022; 4,163,860; 3,907,868; and 3,257,335.

US-A-4,163,860 discusses the use of magnesium hydroxide, as a catalyst to convert monomer to dimethyl terephthalate (DMT) in a two-step extended reaction time process at elevated temperatures. French Patent 1,081,681 presents a process where PET is first glycolized using ethylene glycol to form low molecular weight oligomers and monomer. The reaction products are then dissolved in methanol. Subsequently, sodium methylate is added to promote the reaction. These disclosures are for a process for recovery of PET.

PEN is a polycondensation polymer and, therefore, the molecular weight of the polymer can be reduced by reaction with a monohydric of dihydric alcohol at appropriate reaction conditions. The crude products then can be refined by methods such as distillation and crystallization to product starting materials of suitable purity. Recovery of polyesters utilizes the chemistry of the polyester reaction. Synthesis and depolymerization paths are controlled by equilibrium considerations due to the reversibility of the reactions.

A linear polyester is prepared by reacting a difunctional organic acid and a difunctional alcohol. In some cases it is convenient to substitute the diester of the difunctional acid (for instance dimethyl 2,6 naphthalene dicarboxylate for the naphthalene 2,6 dicarboxylic acid). Usually the reaction is carried out at elevated temperature in the presence of an appropriate catalyst. Reaction by-products are removed from the reactor to drive the equilibrium in a direction that will cause the molecular weight of the polyester to increase.

In polyester scrap recovery, selected reaction by-products are added to the reactor in order to decrease the molecular weight of the polyester by depolymerization to monomer components. The diester form of recovered monomer is preferred, since the diester is more volatile than the acid and is more readily purified.

The generic polyester reaction can be written as two steps:
Step 1: Transesterification
Dimethyl Ester + Glycol = Half Ester + Monomer + Methanol

   CH₃-A-CH₃ + HO-G-OH = CH₃-A-G-OH + HO-G-A-G-OH + CH₃-OH
Step 2: Polycondensation
Monomer = Polymer + Glycol

   HO-G-A-G-OH = HO-(G-A)ₓ-G-OH + HO-G-OH
wherein:
A is a difunctional acid moiety (-COO-T-COO-)
T is benzene for terephthalate and naphthalene for NDC
G is an alkylene moiety (-CH₂-CH₂- for ethylene glycol)
x is the number of repeat units in an average molecular chain, that is, the degree of polymerization.

In the transesterification step the difunctional acid can be substituted for the diester and the reaction product will be water. Likewise other alcohols may be substituted for methanol to form the diester.

In polyester scrap recovery the above reactions are driven in the reverse direction, under appropriate reaction conditions and in the presence of catalysts, by addition of excess amounts of alcohol, glycol or both. Reacting the polymer with excess glycol will reverse the polycondensation reaction and will produce monomer (one diacid moiety with two glycols attached.) Other oligomers will be also present, as determined by the equilibrium conditions for the particular reaction. Reacting alcohol with monomer will then produce the difunctional ester product. Similar results will be obtained when excess alcohol (such as methanol) is reacted with the polymer. The final composition will again be governed by equilibrium. All the major components shown in the transesterification reaction, and a distribution of higher oligomers, are the reaction products.

The present invention provides a process for the depolymerizing of scrap polyethylene naphthalate polyester to monomer components that then can be used to make additional polyester.

A process has been discovered so that PEN can be readily recovered using a two-step procedure. First the high molecular weight polymer is depolymerized to a monomer using glycols at elevated temperature. Then the monomer is converted to the diester of 2,6 naphthalene dicarboxylate by reaction with a monohydric alcohol that was treated with an alkali metal hydroxide at relatively mild reaction conditions. The reaction conditions are critical in order to produce crystals that can be readily washed to remove contaminants from the product.

This process, and variations thereof, provide advantageous means for recovering monomer components from PEN polyester. The benefits and advantages of the processes will be apparent from the detailed discussion of the process below.

Figure 1 is a schematic of a batch reaction wherein caustic alcohol is added to a reaction containing crude polyester and a glycol.

Figure 2 is a schematic of a two reaction batch process.

Figure 3 is a schematic of a semi batch reaction.

Figure 4 is a schematic of a continuous feed reaction process.

For a better understanding of the present invention, together with other and further objects, advantages and capabilities thereof, reference is made to the following detailed description and appended claims in connection with the preceding drawings and description of some aspects of the invention.

Although the polyester chemistry described above is well understood, implementing it in a practical recovery processes is not simple. Complex and sometimes interacting steps of heat and mass transfer must be considered. Some of the required processing steps include heat transfer, transport of two-phase or three-phase systems, crystallization, filtration, distillation, storage, as well as disposal of contaminants and reaction byproducts. Moreover, the physical properties of PEN and NDC differ markedly from PET and DMT. Therefore, experience in recovering DMT from PET is not directly translatable to recovery of NDC from PEN. For example, reactor design and process conditions required to recovery PEN will be different than those for PET. Physical properties of the polyester must be taken into consideration in determining suitable recovery conditions for PEN. Table 1a compares selected physical properties of the two polyesters.

**Table 1a -**

| Physical Properties of PEN and PET | | | | |
|---|---|---|---|---|
| | PET | PEN | DMT | NDC |
| Melting Point, °C | 250 | 265 | 140 | 190 |
| Tg, °C | 69 | 113 | N/A | N/A |
| Sol. in MeOH, weight % at 25°C | 0 | 0 | 1.0 | <0.1 |

The volatility of DMT and NDC are different. This impacts not only the performance of the recovery processes but also influences the ease or difficulty of refining the reaction products.

In the present invention the caustic methanolysis step is used to treat a process stream to improve the yield and to change the properties of the waste stream.

The glycolysis reaction to reduce the molecular weight of PEN scrap can be carried out in several ways. Due to the refractory nature of PEN glycolysis must be carried out at temperatures above the ambient pressure boiling point of ethylene glycol (196°C). This can be accomplished by reacting the PEN and glycol such as ethylene glycol is a pressurized reactor where temperatures of 210° to 280°C, preferably 230° to 240°C can be obtained. Generally, pressures of above one atmosphere are used. An alternative glycolysis procedure can be carried out at ambient pressure using higher boiling point glycols such as diethylene glycol, triethylene glycol or a mixture of glycols that produce the required boiling point to react PEN at ambient pressure in a reasonable length of time.

Polyester monomers can be converted to the diester form at relatively mild conditions by reaction with a monohydric alcohol such as methanol, that has been treated with a hydroxide of an alkali metal such as sodium, potassium, lithium and others. If desired other monohydric alcohols can be substituted for methanol including ethanol, propanol and others. The object is to produce a diester that has the lowest possible boiling point (highest volatility) to affect refining by distillation.

The reaction paths for methanol are shown below:
1. Methanol reaction

   NAOH + CH₃OH = NaOCH₃ + H₂O (1)
2. Diester formation

   2 NaOCH₃ + HO-G-A-G-OH = 2 NaO-G-OH + CH₃-A-CH₃ (2)
where A is the difunctional acid group (naphthalene group)
G is the glycol group (-CH₂-CH₂- for EG-CH₂-CH₂-CH₂- for DEG and so forth)

Reaction (2) can be most conveniently carried out from room temperature and up to the boiling point of the monohydric alcohol used. The methanolysis reaction step consists of a two phase system at a reaction conditions. The product of the reaction (2) is a slurry of NDC crystals in methanol and glycol. In order to obtain a practical process the conditions must be closely controlled to produce crystal size and size distribution that will have good permeability for washing and separation. To run the methanolysis reaction above the boiling point of the alcohol a pressurized reactor is required.

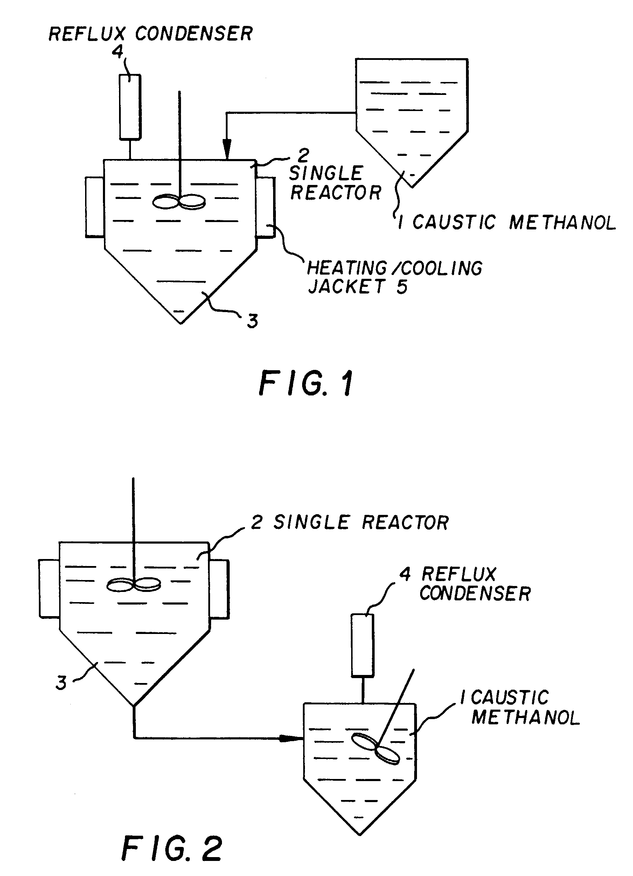

Figures 1 through 4 show examples of how the invention can be implemented in recovery of PEN polyester scrap.

Figure 1 is a schematic representation of a reactor system utilizing a single reactor 2. The PEN polyester scrap is first reacted with a glycol 3 in the reactor to form monomer. Caustic methanol is prepared by dissolving an alkali metal hydroxide in methanol in a separate tank 1. The caustic methanol is then added to the monomer to complete the process. The resulting slurry is then removed from the reactor for further processing. The reaction cycle then is repeated. In Figure 1, a heating/cooling jacket 5 is used to control the temperature and a reflux condenser 4 removes the excess heat from the reactor.

Figure 2 shows a two reactor batch process where the glycolysis is carried out in the first vessel 2 and the monomer 3 is then transferred to a second reactor 1 where the second reaction step is carried out. This allows the addition of the caustic methanol to the monomer, addition of monomer to the caustic methanol or mixing the two streams in the second reactor at the same time. A reflux condenser 4 removes excess heat from the reactor.

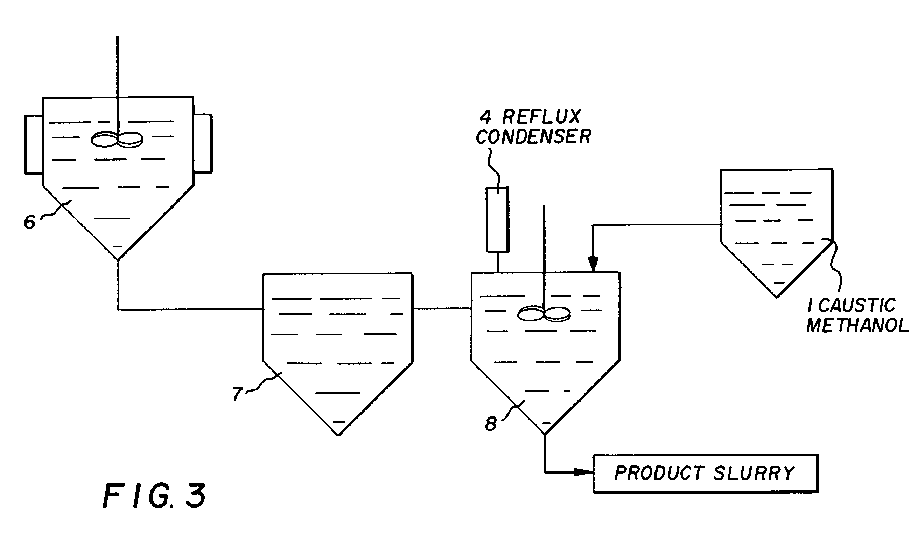

Figure 3 shows a semibatch reactor system where the monomer is produced batchwise in the first reactor 6 and then transferred to a holding tank 7. The monomer 3 then is continuously formed to the second stirred tank reactor 8 where the monomer is mixed with a stream of caustic methanol from tank 1. The product slurry is continuously removed from the second reactor for further processing and a reflux condenser 4 removes excess heat from the reactor.

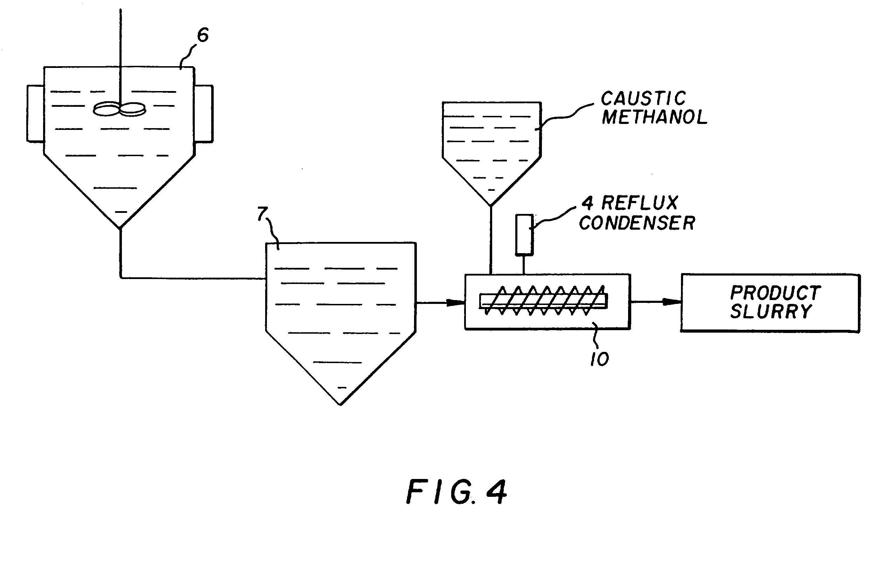

Figure 4 presents a reactor system similar to the one shown in Figure 3 except that an augered plug flow reactor 10 is used to perform the second reaction step.

The process can also be run in a completely continuous manner where the first reactor is a continuous stirred reactor. The scrap and glycol are continuously added to the reactor and the monomer is continuously removed from the reactor and sent to the methanolysis reactor of chosen design for the second reaction step.

The embodiment of the invention is illustrated in the examples.

Caustic methanolysis experiments were performed using conventional laboratory round bottom flasks. The glycolysis step was carried out using DEG in a glass reactor equipped with a reflux condenser and an agitator. Diethylene glycol was used with PEN to obtain high enough reaction temperature without pressurization. A molten salt bath was used to heat the reactor. The salt bath temperature was maintained at 295°C providing sufficient temperature gradient to maintain boiling of the glycol.

The caustic methanolysis step was also carried out in a glass flask equipped with a heated dropping funnel, a water cooled reflux condenser and an agitator. This reactor was not heated.

A required charge of ground PEN scrap was added to the round bottom flask. The DEG and zinc acetate catalyst were then introduced into the reactor. To obtain the needed heat input the reactor was immersed in the molten salt to a level where DEG was boiling and some condensate was evident in the reflux condenser. The reaction was run until all the solids were in solution (4 hours).

The caustic methanol was prepared by adding the required amount of sodium hydroxide pellets to the methanol. The mixture was stirred until all the sodium hydroxide reacted and a clear solution was obtained. The temperature of the caustic methanol at these conditions generally the sodium hydroxide is added at from room temperature to the boiling point of methanol. The caustic methanol contains from 0.5 to 5 weight percent sodium hydroxide was between 25 and 27°C.

The methanolysis step consisted of introducing the molten monomer produced in the glycolysis step into the flask containing the required amount of caustic methanol using a dropping funnel. To maintain uniform temperature the mixture was constantly agitated. Since molten monomer was at 200 to 250°C some methanol flashing occurred. The excess heat was removed by the reflux condenser. The reactor was at ambient pressure. The reaction mixture temperature stabilized at the boiling point of methanol (64°C). The slurry formed by the reaction was cooled at room temperature and the liquid and solids were separated by filtration. The NDC product was washed with fresh methanol to remove the residual ethylene glycol and sodium containing components.

Table 1 shows the experimental conditions and results for the two step caustic methanolysis process. The effect of the amount of DEG in the glycolysis step as well as the ratio of caustic methanol to polymer ratios were evaluated. The quality and composition of the product DMT was determined by analysis and by measuring filtration quality. The filtration times listed in the table were obtained using a Buechner funnel with No. 42 filter paper. The filtration was carried out using 22 inches Hg of vacuum.

The caustic methanolysis weight ratios were defined as follows:
Methanol/Polymer - based on the weight of the original polymer used in the glycolysis step.
Methanol/Monomer - based on the monomer produced by glycolysis (this includes the free glycol content).

**Table 1**

| Caustic Methanolysis Process | | | | | | | |
|---|---|---|---|---|---|---|---|
| Glycolysis Using DEG | | | Caustic Methanolysis | | | | |
| | | | Weight Ratios | | Results | | |
| Scrap, grams | DEG grams | Mole Ratio | Methanol/ Polymer | Methanol/ Monomer | NDC % | Filtration Quality | Time, second |
| 392 | 200 | 1.2:1 | 2.3 | 1.5 | 44.4 | Poor | >300 |
| 396 | 272 | 1.6:1 | 2.5 | 1.5 | 65.3 | Poor | >300 |
| 396 | 340 | 2:1 | 2.8 | 1.5 | 81 | Fair | 160 |
| 386 | 680 | 4:1 | 2.7 | 2 | 97 | Good | 60 |
| 386 | 680 | 4:1 | 4:1 | 1.5 | 98 | Good | 55 |
| 386 | 680 | 4:1 | 5.4 | 1 | 98 | Good | 60 |
| 386 | 680 | 4:1 | 4. | 1.5 | 98-99 | Very Good | 25 |
| Reaction Time - 4 Hr. NaOH in Methanol - 1.5% | | | | | | | |
| Reaction Temperature - BP of the glycol | | | | | | | |

The NDC concentrations presented in the Table are for crude product. Purities of 98 to 99% were possible with a two stage methanol was with product having desirable crystal size distribution and good permeability.

The examples presented above are for a batch recovery process. The reactions steps can be also accomplished in a semibatch or a continuous manner. For instance, the glycolysis reaction can be carried out in a batch reactor and the molten product can be stored in a holding tank. The monomer then can be continuously fed into continuous stirred tank reactor or a plug flow reactor equipped with appropriate agitation for the methanolysis step. Likewise all the steps can be carried out in continuous reactors. Due to relatively mild reaction conditions simple reactors of conventional design can be used.

It is seen from the above that a new convenient process for recovering scrap PEN, which requires only mild reaction conditions and a single reaction schedule is developed wherein the caustic method comprises 0.5 to 5 weight percent sodium hydroxide, the DEG to scrap molar ratio is 2:1 to 10:1, the caustic methanol to scrap ratio is 2 to 1 to 5 to 1, the glycolysis temperature is from 230 to 260°C and the methanolysis temperature is from room temperature to the boiling point of alcohol.

## Claims

1. A process for recovering monomer components from polyethylene naphthalate polyester, the process comprising the steps of:
a) preparing caustic monohydric alcohol by adding sodium hydroxide to monohydric alcohol at from room temperature to the boiling point of alcohol and wherein the caustic alcohol contains from 0.5 to 5 weight percent alkali metal hydroxide, and
b) subsequently reacting polyethylene naphthalate scrap polyester with a glycol and the caustic alcohol from step a) at a diethylene glycol to scrap polyester molar ratio of 2 to 1 to 10 to 1 and a caustic alcohol to scrap polyester ratio of 2:1 to 5:1 and at a temperature of 230°C to 260°C.

2. The process of claim 1 wherein the alkali metal hydroxide is sodium hydroxide.

3. The process of claim 1 wherein the caustic alcohol is caustic methanol.

4. The process of claim 1 wherein the diethylene glycol to scrap polyester ratio is 2:1.

5. The process of claim 1 wherein the caustic methanol to polyester scrap is 4.1:1.

6. The process of claim 1 wherein the glycolysis temperature is 210°C to 280°C.

7. The process of claim 1 wherein the scrap polyester and diethylene glycol are added to a reactor containing the caustic alcohol from step a).

8. The process of claim 1 wherein step a) and step b) are carried out in batch mode.

9. The process of claim 1 wherein step a) is carried out in batch mode and step b) is carried out in a continuous mode.

10. The process of claim 1 wherein both step a) and step b) are carried out in a continuous mode.
